Europäisches Patentamt

European Patent Office

Office européen des brevets

(19)

(11) **EP 0 893 966 B1**

(12) **EUROPÄISCHE PATENTSCHRIFT**

(45) Veröffentlichungstag und Bekanntmachung des Hinweises auf die Patenterteilung:
**04.04.2001 Patentblatt 2001/14**

(51) Int Cl.$^7$: **A61B 5/0496**, A61B 5/0484

(21) Anmeldenummer: **97949978.7**

(22) Anmeldetag: **29.11.1997**

(86) Internationale Anmeldenummer:
**PCT/DE97/02791**

(87) Internationale Veröffentlichungsnummer:
**WO 98/24364 (11.06.1998 Gazette 1998/23)**

(54) **VERFAHREN UND ANORDNUNG ZUR BESTIMMUNG DER TOPOGRAPHIE FÜR REAKTIONSSIGNALE EINES AUGES**

PROCESS AND SYSTEM FOR DETERMINING THE TOPOGRAPHY OF EYE REACTION SIGNALS

PROCEDE ET SYSTEME POUR DETERMINER LA TOPOGRAPHIE DES SIGNAUX DE REACTION D'UN OEIL

(84) Benannte Vertragsstaaten:
**AT BE CH DK ES FI FR GB GR IE IT LI LU MC NL PT SE**

(30) Priorität: **02.12.1996 DE 19649858**

(43) Veröffentlichungstag der Anmeldung:
**03.02.1999 Patentblatt 1999/05**

(73) Patentinhaber: **Cindatec Ingenieurtechnische Dienste GmbH**
**09125 Chemnitz (DE)**

(72) Erfinder:
• **KUTSCHBACH, Ernst Dr.**
**D-09119 Chemnitz (DE)**
• **HELD, Uwe Dr.**
**D-09126 Chemnitz (DE)**
• **HOCH, Uwe**
**D-09355 Gersdorf (DE)**
• **GÜNTHER, Ralf**
**D-08393 Meerane (DE)**
• **STASCHE, Manfred**
**D-65197 Wiesbaden (DE)**

• **HINKEL, Albrecht**
**D-09573 Augustusburg (DE)**

(74) Vertreter: **Seerig & Hübner**
**Patentanwälte**
**Am Alten Bad 6**
**09111 Chemnitz (DE)**

(56) Entgegenhaltungen:
**WO-A-92/03088 US-A- 5 539 482**

• **B. BROWN ET AL.: "Contrast and luminance as parameters defining the output of the VERIS topographical ERG" OPHTHALMIC & PHYSIOLOGICAL OPTICS , Bd. 16, Nr. 1, Januar 1996, Seiten 42-48, XP002062775**
• **E.E. SUTTER ET AL.: "The Field Topography of ERG Components in Man" VISION RESEARCH, Bd. 32, Nr. 3, 1992, Seiten 433-446, XP002062776 in der Anmeldung erwähnt**
• **S. PARKS ET AL.: "Comparison of repeatability of the multifocal electroretinogram and Humphrey perimeter" DOCUMENTA OPHTHALMOLOGICA, Bd. 92, 1997, Seiten 281-289, XP002062777**

## Beschreibung

Die Erfindung betrifft ein Verfahren und eine Anordnung zur Bestimmung der Topographie für Reaktionssignale eines Auges, bei dem zur Stimulierung auf einer vor dem Auge angeordneten Fläche ein aus Teilflächen bestehendes leuchtendes Bild dargestellt wird, wobei jede Teilfläche durch eine ihr zugeordnete digitale Zeitfunktion hell- und dunkelgesteuert und dabei die Gesamtreaktion des Auges gemessen wird.

Die ermittelte Topographie für die Reaktionssignale eines Auges zeigt die objektive Empfindlichkeitsverteilung der Netzhaut und gibt somit Aufschluß über das Sehvermögen. Mit einer solchen Untersuchung ist eine Früherkennung und Bewertung von Augenkrankheiten z.B. des Glaukom möglich. Vor allem sind mit derartigen Untersuchungen partielle Fehler der Netzhaut feststellbar.

Es gibt eine Vielzahl von Untersuchungsmethoden, die auf subjektiver Basis arbeiten, d.h. bei denen der untersuchte Patient die Messung durch seine Aussage bewertet. Bei allen diesen Methoden ist der Patient in die Messung integriert, indem er die Aussage gibt, ob und wie er einen bestimmten Reiz wahrnimmt.

Als objektives Meßverfahren hat sich das Elektroretinigramm (ERG) etabliert, bei dem das mit einer Elektrode vom Auge abgenommene Reaktionssignal mit seinem zeitlichen Verlauf dargestellt und ausgewertet wird. Zur Stimulierung werden einmalige Lichtblitze oder Hell-Dunkelfolgen (Flicker-ERG) verwendet. Dabei wird der Mittelwert für die gesamte Netzhautfläche bestimmt. Bei der Aufnahme evozierter Potentiale werden Elektroden an bestimmter Stelle des Kopfes angebracht, und das gemessene Signal entspricht der Reaktion, die über Nervenstränge an der Meßposition auftritt. Einzelheiten hierzu sind von J. Jörg und H. Hielscher in dem Buch "Evozierte Potentiale in Klinik und Praxis"/Springer Verlag veröffentlicht.

Zur Bestimmung der Topologie der Netzhautempfindlichkeit besteht grundsätzlich die Möglichkeit, Teilflächen der Netzhaut durch einzelne der jeweiligen Teilfläche zugeordnete Lichtreize zu stimulieren und die Reaktion zu messen. Da zur Reduzierung von Meßfehlern Mittelwerte gebildet werden müssen, ergibt sich bei diesem Verfahren eine unvertretbar lange Meßzeit.

In der Patentschrift US 5 382 987 wird die Kopplung einer ophtalmoskopischen Anordnung auf Basis eines 3-Wege Maxwell Betrachtungssystems zur optischen Untersuchung der Netzhaut des Auges mit einer perimetrischen Anordnung zur Bestimmung des Gesichtsfeldes vorgeschlagen, wobei die spektrale Empfindlichkeit eines ausgewählten Teiles der Netzhaut mit Hilfe des Elektroretinogrammes gemessen werden kann, für das ein Stimulusmuster auf die Netzhaut übertragen wird. Mit diesem Verfahren kann die Empfindlichkeit eines einzelnes Teiles sowie das Gesamtbild der Netzhaut untersucht werden. Für die meßtechnische Untersuchung aller Teile der Netzhaut würde sich auch hier eine unvertretbar lange Untersuchungszeit ergeben.

Die Verwendung eines mit einem optischen Modulator versehenen Laser Projektors zur Erzeugung eines Helligkeitsmusters auf der Netzhaut für die Messung des Muster-Elektroretinogramms (PERG) ist von Daniel R. Peters und John Tabora in der Patentschrift US 5 233 373 angegeben worden. Damit kann aber auch nur immer eine ausgewählte Fläche der Netzhaut untersucht werden und die Untersuchung mehrerer Flächen kann nur nacheinander erfolgen.

Ein verbessertes Verfahren und die zugehörige Anordnung zur Bestimmung der Funktionsverteilung der Reaktion über die Fläche der Netzhaut auf Reize ist von R. Richardson in der Patentschrift EP 0 375 737 angegeben worden. Hierbei wird die Gesamtreaktion der Netzhaut auf Stimuli im Sehfeld aufgenommen, wobei die Stimuli durch Serien von Mustern gebildet werden, deren Intensität sich in horizontaler Richtung und in vertikaler Richtung ändert. Als Beispiel wird eine sinus- bzw. cosinusförmige Verteilung der Intensität verwendet, und durch Rücktransformation aus den gemessenen Gesamtsignalen läßt sich die Empfindlichkeitsverteilung über die Fläche der Netzhaut berechnen. Nachteilig bei diesem Verfahren ist, daß sich Meßfehler bei der Bestimmung der einzelnen Koeffizienten nicht erkennen lassen, daß sich aber einzelne Meßfehler auf die Berechnung der gesamten Verteilungsfunktion auswirken. Nachteilig ist bei diesem Verfahren weiterhin, daß die Auflösung auf die größte Dichte der Empfindlichkeitsverteilung angepaßt werden muß, obwohl diese nur in einem eng begrenzten Bereich vorhanden ist.

In der Patentschrift US 5 539 482 wird ein Verfahren angegeben, bei dem das Stimulusbild aus mehreren Vierecken mit vom Zentrum nach außen zunehmender Größe besteht, deren Helligkeitsverlauf mit unterschiedlichen Frequenzen im Bereich von 10 Hz bis 45 Hz gesteuert wird. In dem dargestellten Beispiel wird mit 9 gleichzeitig in der Helligkeit modulierten Vierecken gearbeitet und die Auswertung des gemessenen Signals erfolgt mit Hilfe der Fouriertransformation. Der Vorteil des Verfahrens liegt darin, daß durch Messung der Nyquistfrequenz Einflüsse der unteren Ganglienzellschichten erfaßt werden können. Nachteilig ist, daß zur Bestimmung der Nyquistfrequenz mehrere Messungen mit unterschiedlicher Verteilungen der Modulationsfrequenzen durchgeführt werden müssen und daß die Meßzeiten für jede Frequenzverteilung so lang gewählt werden müssen, daß eindeutige Ergebnisse für die einzelnen Frequenzen ermittelt werden können. Es wird angegeben, daß eine Erweiterung bis auf 32 "Zonen" möglich ist. Damit hat das Verfahren selbst bei dieser Erweiterung noch eine sehr niedrige Auflösung hinsichtlich der untersuchbaren Teilflächen.

Ein anderes Verfahren wurde von E. E. Sutter und D. Tran in der Zeitschrift Vision Research (Great Britain) Vol. 32, No. 3, pp 433-446, 1992 angegeben, mit dem relativ gute Ergebnisse erzielt worden sind. Zur Bestimmung der Topographie der ERG Komponenten wird ein digitales Verfahren angewendet, bei dem als Stimuli Sechsecke verwendet werden, deren zeitliche Helligkeitsverläufe durch m-Sequenzen gesteuert werden. Dabei werden 241 Sechsecke verwendet, deren Größe vom Mittelpunkt aus nach außen zunimmt. Damit wird der ungleichen Dichte der Verteilungsfunktion Rechnung getragen. Es wird das Gesamtreaktionssignal des Auges gemessen und durch Berechnung der Kreuzkorrelationsfunktion mit der jeweiligen m-Sequenz wird der Signalverlauf für das betreffende Sechseck berechnet. Durch Wichtung des Signalverlaufes mit dem Mittelwert für den entsprechenden Bereich von Teilflächen werden Störungen bei der Bestimmung der in dem Signalverlauf enthaltenen Amplitude des Nutzsignales reduziert. Für die Messung werden m-Sequenzen mit einer Länge von 65535 Schritten verwendet, die gegeneinander immer um 256 Schritte versetzt sind. Bei der Bildwechselfrequenz von 67 Hz ergibt sich eine Gesamtmeßdauer von etwa 16 Minuten, wobei die Messung in 32 zeitliche Abschnitte unterteilt wurde, die jeweils 30 Sekunden zuzüglich einer Zeit für die Überlappung betragen. Ein erster Nachteil ist, daß das Verfahren nur in dem Umfang exakte Ergebnisse liefern kann, wie die Reaktionsfunktion der Netzhaut auch linear auf die Reize reagiert. Dieser Zusammenhang ist aber bei einer Bildwiederholfrequenz von 67 Hz nur teilweise und dann auch nur bei kurzer Nachleuchtdauer des Bildschirmes gegeben. Ein weiterer Nachteil dieses Verfahrens besteht darin, daß der Signalverlauf subjektiv überwacht werden muß und bei erkennbaren Störungen z.B. durch Lidschlag oder bei Kontaktproblemen der Elektrode der zugehörige Zeitabschnitt zu wiederholen ist. Der wesentlichste Nachteil ist aber, daß es keine Möglichkeit gibt, die Zwischenergebnisse der einzelnen Zeitabschnitte zu beurteilen und daß erst am Ende der Messung, d.h. nach Vorliegen der Meßwerte über alle Zeitabschnitte die Möglichkeit besteht ein Ergebnis zu ermitteln und dieses Ergebnis zu bewerten, so daß bei nicht erkannten Störungen die gesamte Messung zu wiederholen ist.

In der Amerikanischen Patentschrift US 4 846 567 wurde von E.E. Sutter bereits ein Grundprinzip des Verfahrens angegeben, bei dem als Stimuli ein Displays mit einem quadratischen Array von aktivierbaren Elementen verwendet wird, wobei die zeitlichen Helligkeitsverläufe der Elemente von m-Sequenzen gesteuert werden. Die Berechnung der einzelnen Reaktionssignale erfolgt mit Hilfe der Kreuzkorrelationsfunktion. Auch hier werden m-Sequenzen der Länge $2^{16} - 1 = 65535$ angewendet, die jeweils um 256 Schritte gegeneinander versetzt sind. Ebenso wird vorgeschlagen, die gesamte Messung in zeitliche Abschnitte von etwa 20 bis 40 Sekunden zu unterteilen. Damit entspricht das Prinzip in allen wesentlichen Punkten dem Verfahren, wie es in der Zeitschrift Vision Research benutzt wird und es sind die gleichen Nachteile enthalten, wie in der genannten Veröffentlichung.

Der Erfindung liegt die Aufgabe zugrunde, ein Verfahren und eine Anordnung zu schaffen, mit der die Topographie der Netzhautreaktionen mit hoher Effektivität und großer Sicherheit ermittelt werden kann, wobei jedes Zwischenergebnisses bewertbar ist, um nur solche Zwischenergebnisse in die Rechnung einzubeziehen, die als qualitativ gut erkannt werden damit sowohl das Bedienpersonal als auch die Patienten so gering wie möglich belastet werden und wobei das Verfahren und die Anordnung auch speziellen Untersuchungen angepaßt werden können.

Erfindungsgemäß wird die Aufgabe durch die in den Ansprüchen 1 und 15 genannten Merkmale gelöst. Vorteilhafte Ausgestaltungen sind in den Unteransprüchen angegeben.

Der Vorteil der erfindungsgemäßen Lösung besteht darin, daß für die Messung gleiche, in sich abgeschlossene zeitliche Abschnitte verwendet werden und daß die Qualität des ermittelten Teilergebnisses für jeden zeitlichen Abschnitt bewertet werden kann, so daß nur als gut befundene Teilergebnisse für die weitere Auswertung verwendet werden. Aufgetretene Meßfehler können sofort erkannt werden und es ist nicht erforderlich, die gesamte, aus mehreren zeitlichen Abschnitten bestehende Messung zu wiederholen, sondern es muß nur ein weiteres Teilergebnis durch einen zeitlichen Abschnitt ermittelt werden. Da immer mit gleichen zeitlichen Abschnitten gearbeitet wird, kann deren Anzahl beliebig gewählt werden, d.h. bei auftretenden Unsicherheiten kann die Genauigkeit durch Hinzufügen weiterer zeitlicher Abschnitte verbessert werden. Vorteilhaft ist weiterhin, daß das Signal für die Gesamtreaktion des Auges auf Grenzwerte überwacht wird und bei auftretenden Überschreitungen die m-Sequenzen um eine vorgebbare Anzahl von Schritten zurückgesetzt und die Signale für die Gesamtreaktion des Auges durch die wiederholten Werte ersetzt werden.

Mit der einstellbaren Dauer für den ersten Teilschritt, in dem das durch die m-Sequenz definierte Bild angezeigt wird, und den zweiten Teilschritt, in dem die Fläche dunkelgesteuert ist, kann die Anordnung für die Messung ausgewählter Reaktionen der Netzhaut des Auges angepaßt werden.

Die erfindungsgemäße Lösung soll anhand eines Ausführungsbeispieles näher erläutert werden. In der dazugehörigen Zeichnung zeigen:

Fig. 1     den Prinzipaufbau der erfindungsgemäßen Anordnung,
Fig. 2     ein aus Sechsecken bestehendes Bild mit 61 Teilflächen,
Fig. 3     ein aus Vierecken bestehendes Bild mit 61 Teilflächen,
Fig. 4     ein aus Kreisringsegmenten bestehendes Bild mit 61 Teilflächen,
Fig. 5     Signalverlauf verlängerter m-Sequenzen mit der Länge L' = 8 und
Fig. 6     Zuordnung der Teilflächen zu den Summensignalen.

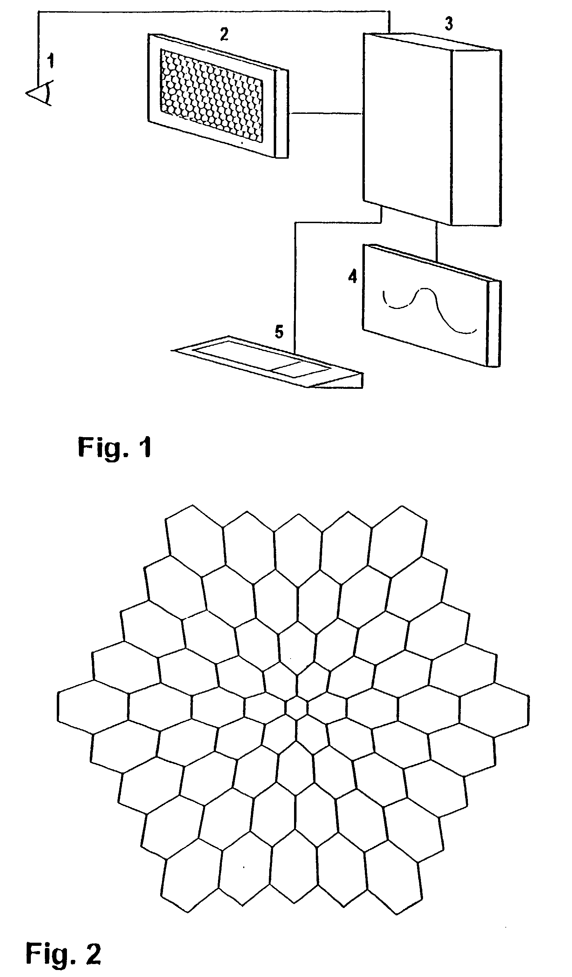

In Fig. 1 ist der Prinzipaufbau der für die Messung verwendeten Anordnung dargestellt. Ein Auge 1 des Patienten blickt auf eine Fläche 2, auf der das leuchtende Bild dargestellt wird, das aus einer Anzahl N Teilflächen besteht. Die Hell- und Dunkelsteuerung der Teilflächen erfolgt über eine Steuereinheit 3. Die Bedienung, d.h. die Einstellung der Meßparameter, erfolgt über eine mit der Steuereinheit 3 verbundene Tastatur 5. Über eine mit der Steuereinheit 3 verbundene Anzeigeeinheit 4 erfolgt die Bedienerführung und die Ergebnisanzeige. Das am Auge 1 abgenommene Reaktionssignal, das sich aus der Summe der Einzelreaktionen aller Teilflächen zusammensetzt, wird über einen Verstärker 6 mit einstellbarer oberer und unterer Grenzfrequenz und eine synchron zur Bilddarstellung gesteuerte Abtastschaltung einem Analog-/Digital-Umsetzer zugeführt, dessen Ausgangsanschlüsse mit der Steuereinheit 3 für die Weiterverarbeitung verbunden sind. Die Einstellung der Parameter des Verstärkers erfolgt über die Tastatur 5 und die Steuereinheit 3. In der Steuereinheit 3 wird der Zeitverlauf des Reaktionssignals gespeichert und kann über die Anzeigeeinheit 4 dargestellt werden. Nach der Berechnung der Reaktionssignale der Teilflächen können diese ebenfalls über die Anzeigeeinheit 4 einzeln oder als Funktion über mehrere Teilflächen angezeigt und deren Amplituden berechnet werden.

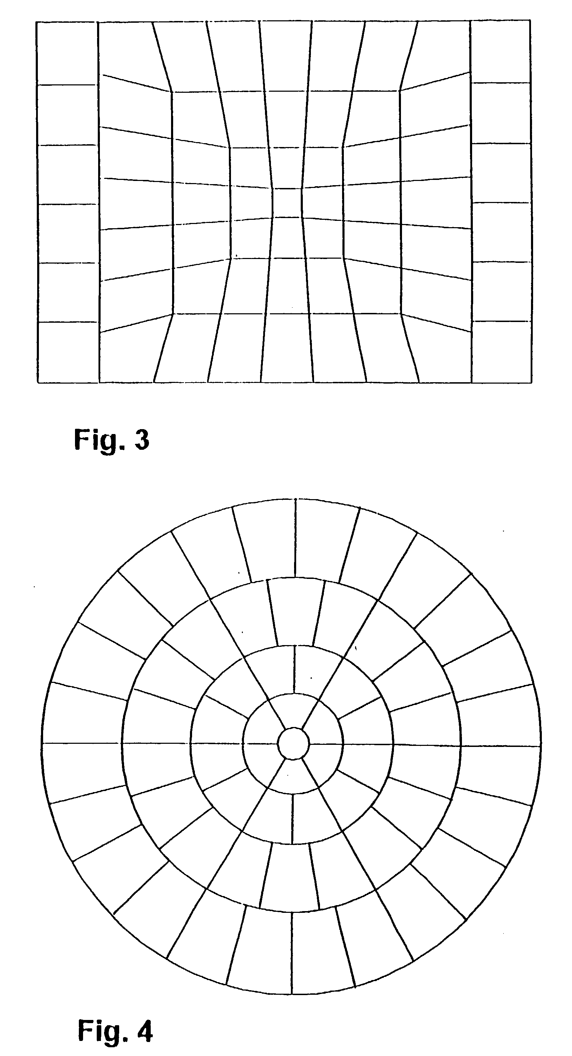

Auf der Fläche 2 werden Bilder dargestellt, die aus abgegrenzten Teilflächen mit von der Mitte nach außen zunehmender Größe bestehen. Als Beispiel mit N = 61 Teilflächen zeigt Fig. 2 ein aus Sechsecken, Fig. 3 ein aus Vierecken und Fig. 4 ein aus Kreisringsegmenten bestehendes Bild.

Die Hell- Dunkelsteuerung der Teilflächen erfolgt durch m-Sequenzen, wodurch sich aus einem einzigen vom Auge abgenommenen Reaktionssignal die Reaktionsfunktionen aller Teilfläche berechnen lassen. m-Sequenzen werden gebildet, indem ein einzelner Koeffizient durch ein irreduzibles, primitives Polynom mittels der binären modulo-2 Division geteilt wird. Bei einem Polynom vom Grad g entsteht damit ein periodisches Ergebnis mit einer Periodenlänge L. Dabei gilt der Zusammenhang zwischen Periodenlänge L und Grad g des Polynoms:

$$L = 2^g - 1 \tag{a}$$

Zur Veranschaulichung soll die Rechnung mit einem Polynom vom Grad g = 3 durchgeführt werden. Wählt man als Beispiel das Polynom

$$P(x) = x^3 + x + 1$$

und für den Koeffizienten $x^{15}$, dann ergibt sich:

$$
\begin{aligned}
x^{15} &: x^3{+}x{+}1 = x^{12}{+}x^{10}{+}x^9{+}x^8{+}x^5{+}x^3{+}x^2{+}x{+}x^{-1}{+}x^{-4}{+}x^{-5}{+}x^{-6}{+} \ldots \\
x^{15}&{+}x^{13}{+}x^{12} \\
&\ \ x^{13} \\
&\ \ x^{13}{+}x^{11}{+}x^{10} \\
&\quad\ x^{12}{+}x^{10}{+}x^9 \\
&\quad\quad x^{11}{+}x^9{+}x^8 \\
&\quad\quad\ \ x^8{+}x^6{+}x^5 \\
&\quad\quad\quad x^6{+}x^4{+}x^3 \\
&\quad\quad\quad\ \ x^5{+}x^4{+}x^3{+}x^2 \\
&\quad\quad\quad\quad x^4{+}x^2{+}x \\
&\quad\quad\quad\quad\ \ x{+}x^{-1}{+}x^{-2} \\
&\quad\quad\quad\quad\quad x^{-1}{+}x^{-3}{+}x^{-4} \\
&\quad\quad\quad\quad\quad\ \ x^{-2}{+}x^{-4}{+}x^{-5} \\
&\quad\quad\quad\quad\quad\quad x^{-3}{+}x^{-5}{+}x^{-6}
\end{aligned}
$$

Stellt man das Ergebnis für alle belegten Koeffizienten mit 1 und für alle unbelegten Koeffizienten mit 0 dar, dann ergibt sich bei Aneinaderreihung der Koeffizienten und unter Beachtung, daß die Koeffizienten $x^{14}$ und $x^{13}$ Null sind:

```
0 0 1 0 1 1 1 0 0 1 0 1 1 1 0 0 1 0 1 1 1 . . . . . . . . . .
_ _ _ _ _ _ _ _ _ _ _ _ _   _ _ _ _ _ _ _ _ _ _ _ _ _   _ _ _ _ _ _ _ _ _ _ _ _ _
   1. Periode        2. Periode        3. Periode
```

Man bezeichnet jeweils eine Periode als m-Sequenz oder Folge maximaler Länge. Von jeder m-Sequenz der Länge L lassen sich genau L um je einen Schritt zyklisch verschobene Folgen bilden, und wenn man die aus den Koeffizienten 0 und 1 bestehende, um i Schritte verschobene m-Sequenz mit $S_i(0,1)$ bezeichnet, dann gilt:

$$S_0(0,1) = 0\ 0\ 1\ 0\ 1\ 1\ 1$$

$$S_1(0,1) = 1\ 0\ 0\ 1\ 0\ 1\ 1$$

$$S_2(0,1) = 1\ 1\ 0\ 0\ 1\ 0\ 1$$

$$S_3(0,1) = 1\ 1\ 1\ 0\ 0\ 1\ 0$$

$$S_4(0,1) = 0\ 1\ 1\ 1\ 0\ 0\ 1$$

$$S_5(0,1) = 1\ 0\ 1\ 1\ 1\ 0\ 0$$

$$S_6(0,1) = 0\ 1\ 0\ 1\ 1\ 1\ 0$$

$$S_7(0,1) = S_0(0,1)$$

Entsprechend ergibt sich die negierte m-Sequenz mit gleichen Eigenschaften:
$/S_0(0,1) = 1\ 1\ 0\ 1\ 0\ 0\ 0$

Für algebraische Berechnungen führt man bei den m-Sequenzen die Substitution
0 -> -1 und 1 -> +1 ein und bezeichnet die damit gebildete negierte m-Sequenz:
$/S_0(-1,+1) = +1\ +1\ -1\ +1\ -1\ -1\ -1$

Bildet man den diskreten Kreuzkorrelationskoeffizienten KKK aus zwei um n Schritte gegeneinander verschobenen m-Sequenzen über alle Schritte s der m-Sequenz mit der Länge L

$$KKK(/S_i, /S_{i+n}) = \frac{1}{L} \sum_{s=0}^{L-1} /S_i(-1,+1) \cdot /S_{i+n}(-1,+1) \qquad \textbf{(b)}$$

so ergibt sich für eine Verschiebung um n = 0 Schritte:
KKK = 1 und für alle anderen Verschiebungen um n ≠ 0 Schritte:
KKK = -1/L
wie sich für das Beispiel mit L = 7 leicht nachvollziehen läßt. Das Ergebnis für n ≠ 0 stellt einen Restfehler dar, den man dadurch eliminieren kann, daß man bei allen m-Sequenzen $/S_i(-1,+1)$ an einer beliebigen aber gleichen Stelle einen Schritt +1 einfügt.

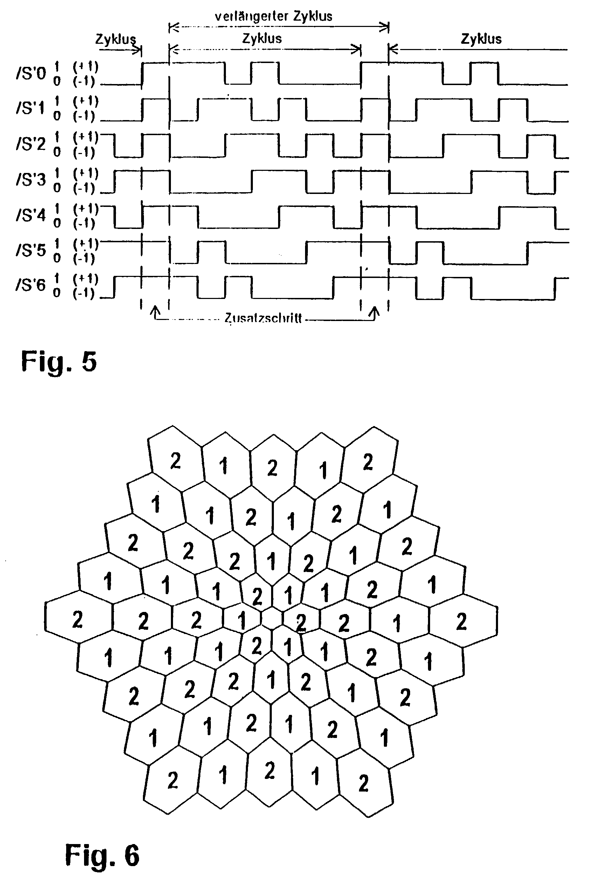

Die so gebildete verlängerte m-Sequenz $/S_i'(-1,+1)$ ist in Fig. 5 dargestellt. Die Kreuzkorrelationskoeffizienten haben für die verlängerten m-Sequenzen die Werte
n = 0:      KKK' = 1          n ≠ 0:      KKF' = 0
wenn man L durch L'=L+1 ersetzt. Für die Länge gilt mit Gleichung (a):

$$L' = 2^g \qquad (c)$$

Werden nun die m-Sequenzen /S$_i$'(0,1) für die Hell-Dunkelsteuerung der Teilflächen benutzt und jede der N Teilflächen erzeugt eine der Helligkeit proportionale Teilreaktion f$_i$(/S$_i$'(0,1)), dann ist das gesamte Reaktionssignal:

$$F(/S) = \sum_{i=0}^{N} f_i(/S_i'(0,1)) \qquad \textbf{(d)}$$

Mit der Kreuzkorrelationsrechnung KKR kann aus diesem Signal nun für jede zur Hell- Dunkelsteuerung benutzte m-Sequenz mit der Verschiebung i die entsprechende Signalkomponente wieder extrahiert werden:

$$KKR'(F(/S),/S_i'(-1,+1)) = \frac{1}{L}\sum_{s=0}^{L-1} F(/S) \cdot /S_i'(-1,+1) = \frac{1}{2}\left(f_i(1) - f_i(0)\right) \quad \textbf{(e)}$$

Das heißt, damit läßt sich aus dem Signalgemisch jede der Teilfunktionen fehlerfrei wiedergewinnen. Das gilt aber nur, wenn es sich um proportionale Abhängigkeiten bei den Funktionen handelt.

Wenn das Reaktionssignal der Teilflächen ein zeitabhängiger, insbesondere von Helligkeitsveränderungen abhängiger Teilfunktionsverlauf $\varphi_i(\tau,/S_i'')$ ist, dann ergibt sich das gesamte Reaktionssignal für N Teilflächen:

$$\Phi(\tau,/S'') = \sum_{i=0}^{N} \varphi_i(\tau,/S_i''(0,1))$$

Wird bei jedem Schritt der m-Sequenzen ein neuer Teilfunktionsverlauf $\varphi_i(\tau,/S_i'')$ ausgelöst, dann können mit Hilfe der Kreuzkorrelationsfunktion KKF die im Gesamtsignal enthaltenen Teilfunktionsverläufe wieder berechnet werden:

$$KKF'(\Phi(\tau,/S''),/S_i''(-1,+1) = \frac{1}{L}\sum_{s=1}^{L-1} \Phi(\tau,/S'')./S_i''(-1,+1) = \frac{1}{2}\varphi_i(\tau,/S_i''(0,1)) \quad \textbf{(f)}$$

Das Ergebnis bleibt auch bei nichtlinearen Zusammenhängen fehlerfrei, wenn die minimale zeitliche Verschiebung $\Delta t$ zwischen den m-Sequenzen größer ist, als die Dauer $\tau_{max}$ des Funktionsverlaufes $\varphi_i(\tau,/S_i'')$, oder wenn sich die ausgelösten Teilreaktionen linear überlagern.

Das Reaktionssignal des Auges ist aber von der Hell- oder Dunkeladaption des Auges sowie der Intensität und der Dauer der Lichtreize abhängig. Die Überlagerung der Signale von mehreren aufeinanderfolgenden Reizen ist erst ab einem bestimmten Zeitabstand zwischen den Reizen etwa linear. Mit der Kreuzkorrelationsfunktion KKF erhält man nur dann ein weitgehend fehlerfreies Ergebnis, wenn die einzelnen Schritte der m-Sequenzen zeitlich so weit gegeneinander verschoben sind, daß die Überlagerung der Signale aufeinanderfolgender Reaktionen nahezu linear erfolgt. Als erste Näherung kann davon ausgegangen werden, daß bei einer mittleren Leuchtdichte dieser Zeitraum etwa $\delta$ = 60 ms beträgt.

Die aus der Kreuzkorrelationsfunktion KKF zurückgewonnene Reaktionsfunktion hat bei N Teilflächen gegenüber der Reaktionsfunktion für die Gesamtfläche bei der Mittelwertsbildung über eine gleiche Anzahl von Meßschritten ein um den Faktor 2N schlechteres Signal-Rausch-Verhältnis. Um das Signal-Rausch-Verhältnis um den Faktor M zu verbessern, müssen M$^2$ Meßschritte durchgeführt werden. Für N = 61 Teilflächen und einen Zeitabstand von etwa $\delta$ = 60 ms Meßschritt ergibt sich eine Meßdauer

$$T_M = M^2 * \delta = 223 \text{ sec} \approx 4 \text{ min,}$$

wenn man M = N setzt.

Über diese Zeitdauer kann aber niemand das Auge absolut ruhighalten. Deshalb wird die Messung in 8 zeitliche Abschnitte von je etwa 30 sec unterteilt und das Gesamtergebnis durch Summierung der Reaktionsfunktionen der Teilflächen über alle zeitlichen Abschnitte gebildet.

Um in jedem zeitlichen Abschnitt die Teilreaktionen mit Gleichung (f) berechnen zu können, enthält jeder Abschnitt eine oder mehrere vollständige verlängerte m-Sequenzen. Für die angesetzten Werte ergibt das eine m-Sequenz mit der Zykluslänge

$$L' = 2^g = 512 \qquad g = 9,$$

wenn man genau eine m-Sequenz für einen zeitlichen Abschnitt vorsieht. Die einzelnen Teilflächen werden durch um jeweils 8 Schritte gegeneinander versetzte m-Sequenzen gesteuert. Damit haben die Zeitfunktionen der Teilflächen einen Abstand von etwa 480 ms gegeneinander.

Durch Veränderung der Einstellung können die Zykluslänge und der Schrittabstand um einen Faktor 2 oder 4 erhöht und gleichzeitig die Schrittdauer entsprechend verringert werden, wodurch sich der Signal-Rausch-Abstand verbessert, aber der Einfluß der Nichtlinearitäten zunimmt. Ebenso können die Zykluslänge um einen Faktor 2 oder 4 verringert und gleichzeitig die Schrittdauer entsprechend erhöht werden, wodurch sich der Einfluß der Nichtlinearitäten reduziert, aber das Signal-Rausch-Verhältnis verschlechtert. Eine Verbesserung ist hier durch eine Erhöhung der Anzahl der zeitlichen Abschnitte möglich.

Jeder Schritt der m-Sequenz besteht aus einem ersten Teilschritt, in dem die Teilflächen entsprechend der m-Sequenzen hell- oder dunkelgesteuert werden und einem zweiten Teilschritt, in dem alle Teilflächen dunkelgesteuert werden. Damit ergibt sich eine eindeutige Anfangsbedingung in jedem Schritt, auch für die Bereiche der m-Sequenz, die aus mehreren aufeinanderfolgenden 1-Schritten bestehen. Die Zeitanteile der beiden Teilschritte sind einstellbar.

Jeder zeitliche Abschnitt besteht aus einem vorgelagerten Teilzyklus, während dem die Einschwingvorgänge der Meßeinrichtung abklingen, einem vollständigen Zyklus für die Ermittlung der Teilergebnisse und einem nachgeordneten Teilzyklus, dessen Dauer mindestens so groß ist, wie die Zeit der durch die Kreuzkorrelation zu berechnenden Reaktionsfunktion.

Nachdem die Messung in einem zeitlichen Abschnitt abgeschlossen ist, erfolgt die Berechnung der Reaktionsfunktionen der Teilflächen nach Gleichung (f). Das Ergebnis aus der Messung eines zeitlichen Abschnittes hat ein sehr schlechtes Signal-Rausch-Verhältnis und kann einzeln kaum ausgewertet werden. Da die Reaktionssignale der Teilflächen aber sehr ähnlich sind, ergibt die Summenfunktion ein wesentlich besseres Signal-Rausch-Verhältnis.

Bildet man zwei Teilsummen entsprechend der Zuordnung der Teilflächen nach Fig. 6, so machen sich Meßfehler und Störungen in den beiden Summenfunktionen durch Unterschiede bemerkbar, da die Anzahl der mit +1 und mit -1 gewichteten Komponenten in den beiden Teilsummen unterschiedlich ist. Durch einen Vergleich der beiden Funktionen miteinander und mit den Ergebnissen vorhergehender bzw. nachfolgender zeitlicher Abschnitte läßt sich die Qualität der Meßergebnisse der einzelnen zeitlichen Abschnitte ermitteln. Für die Auswertung werden nur die als gut erkannten Ergebnisse verwendet, indem die Reaktionsfunktionen der Teilflächen summiert werden. Dabei kann die für die Auswertung verwendete Anzahl der zeitlichen Abschnitte beliebig erhöht werden, da in jedem zeitlichen Abschnitt ein vollständiges Teilergebnis mit schlechtem Signal-Rausch-Verhälnis ermittelt wird, aber durch die Mittelwertbildung verbessert wird.

Zur Darstellung des aus Teilflächen bestehenden Bildes kann eine Bildröhre verwendet werden. In einer anderen Ausführungsvariante wird das Bild auf einer mit LED bestückten Fläche erzeugt. Ebenso kann in einer Ausführungsvariante ein Laserprojektor eingesetzt werden, dessen Strahl für die Bilddarstellung zweidimensional abgelenkt wird. Die flexibelste Zeitsteuerung ist bei der mit LED bestückten Fläche möglich. Sowohl Bildröhre als auch Laserprojektor schreiben das Bild seriell, und die Zeitdauer für die Teilschritte zur Bilddarstellung und den Teilschritt, in dem alle Teilflächen dunkelgesteuert werden, ist nur als ganzzahliges Vielfaches der Bilddarstellungszeit und durch Veränderung der Bildwiederholfrequenz einstellbar. Für die Auswertung der erzeugten Reaktion ist zu beachten, daß der Laserprojektor eigentlich ein ideales serielles Blitzbild erzeugt und eine Bildröhre einen längeren Blitz entsprechend ihrer Nachleuchtdauer. Bei der mit LED bestückten Fläche wird eine definierte Einschaltdauer erzeugt.

Die Reaktionssignale des Auges werden mit einem in oder außerhalb der Steuereinheit 3 angeordneten Differenzverstärker abgenommen, dessen erster Eingang mit einer am oder in unmittelbarer Nähe des Auges angebrachten Elektrode verbunden ist und dessen zweiter Eingang mit einer Elektrode verbunden ist, die an einer elektrisch neutralen Stelle, z.B. der Stirn des Patienten angebracht ist. Das gemessene Signal wird über einen einstellbaren Bandpaß der Steuereinheit 3 zugeführt, um Störsignale außerhalb des zu messenden Frequenzbereiches zu unterdrücken.

Auftretende Störungen, z.B. durch Lidschlag, können durch eine Amplitudenüberwachung erkannt werden, die sowohl auf eine Überschreitung der Amplitude als auch auf eine Unterschreitung der Amplitude über einen bestimmten Zeitraum anspricht. Um auch bei einem solchen Fehler nicht die gesamte Messung zumindest für den betreffenden zeitlichen Abschnitt wiederholen zu müssen, werden die m-Sequenzen unmittelbar nach der Erkennung um eine vorgebbare Anzahl von Schritten zurückgesetzt und die durch die Störung verfälschten Meßwerte werden durch die wiederholten Werte ersetzt. Die Anzahl der rückzusetzenden Schritte r soll r ≥ g sein, wobei g der Grad des Poynoms ist, mit dem die m-Sequenz erzeugt wird.

Für die Messung können auch die Signale des Magnetoretinogrammes oder visuell evozierte Potentiale (VEP) herangezogen werden.

Mit dem dargestellten Verfahren kann die Topologie der Reaktion des Auges auch mit einer höheren Auflösung ermittelt werden, als mit den in Fig. 2, Fig. 3 oder Fig. 4 dargestellten Bildern. In einer ersten Variante wird das Bild in mehrere symmetrisch zum Mittelpunkt liegende Segmente unterteilt und es werden nacheinander die einzelnen Segmente gemessen, wobei jedes Segment etwa 61 Teilflächen hat. Ebenso kann das aus einer größeren Anzahl kleinerer Teilflächen bestehende Bild in mehreren Schritten gemessen werden, indem jeweils unterschiedliche, analog zur Fig. 6 über die Gesamtfläche verteilte Teilflächen für die Messung benutzt werden. Ebenso können Bilder, die aus mehreren kleineren Teilflächen bestehen mit einer zusammenhängenden Messung über einen größeren Zeitraum gemessen werden. Dabei werden entsprechend der Anzahl der Teilflächen längere m-Sequenzen verwendet und die Bewertung der Qualität erfolgt für Gruppen von zusammengehörigen zeitlichen Abschnitten.

Die Steuereinheit 3 besteht aus einem Rechner, der sowohl die digitalen, zyklischen Zeitsignale zur Steuerung des aus Teilflächen bestehenden Bildes erzeugt, als auch die Auswertung der Meßdaten durchführt sowie die Bedienung der Anordnung und die Ergebnisdarstellung steuert. Eine vorteilhafte Ausführungsform ergibt sich, wenn die Steuereinheit 3 aus zwei miteinander gekoppelten Rechnern aufgebaut ist, wobei der erste Rechner die Erzeugung der zyklischen Zeitsignale zur Steuerung des aus Teilflächen bestehenden Bildes und die Auswertung der Meßdaten dient und der zweite Rechner für die Bedienung der Anordnung und die Ergebnisdarstellung verwendet wird.

Der Kern der Erfindung besteht darin, daß für jeden zeitlichen Abschnitt als digitale Zeitfunktion für die Hell- und Dunkelsteuerung der Teilflächen vollständige, vorzugsweise negierte und um einen Schritt verlängerte m-Sequenzen verwendet werden, wodurch für jeden zeitlichen Abschnitt die Reaktionsfunktionen für alle Teilflächen durch Bildung der Kreuzkorrelationsfunktion berechnet werden. Dadurch ist es möglich, die Qualität des Meßergebnisses für jeden zeitlichen Abschnitt zu bewerten und nur gute Messungen in die Bildung des Endergebnisses einzubeziehen.

**Patentansprüche**

1. Verfahren zur Bestimmung der Topographie für bioelektrische Reaktionssignale eines Auges auf Lichtreize in Abhängigkeit vom Ort des jeweiligen Lichtreizes auf der Netzhaut mit einer vor dem Auge angeordneten Fläche, auf der zur Stimulierung ein aus Teilflächen bestehendes leuchtendes Bild dargestellt wird, wobei jede Teilfläche in Abhängigkeit von einer ihr zugeordneten digitalen Zeitfunktion hell- und dunkelgesteuert und dabei die Gesamtreaktion des Auges gemessen wird und bei dem aus der Gesamtreaktion des Auges durch Berechnung der Kreuzkorrelationsfunktion mit der digitalen Zeitfunktion die der jeweiligen Teilfläche zugeordnete Komponente der Reaktionsfunktion des Auges ermittelt wird, dadurch gekennzeichnet, daß für die Messung eine beliebige, über einem Mindestwert liegende Anzahl gleicher, in sich abgeschlossener zeitliche Abschnitte verwendet wird, die jeweils ein bewertbares Teilergebnis liefern, indem für jeden zeitlichen Abschnitt als digitale Zeitfunktion eine vollständige, für jede Teilfläche um mindestens einen Schritt zyklisch verschobene m-Sequenz verwendet wird, in die zu einem beliebigen aber für alle m-Sequenzen gleichen Zeitpunkt ein Zusatzschritt eingefügt wird und für jeden zeitlichen Abschnitt die Reaktionsfunktion jeder Teilfläche durch Bildung der Kreuzkorrelationsfunktion berechnet wird und durch Summierung der Reaktionsfunktionen mehrerer Teilflächen wenigstens eine Summenfunktion gebildet wird, mit denen eine Bewertung der Qualität der Messung des zeitlichen Abschnittes erfolgt und zur Reduzierung der Meßfehler durch Summieren der Reaktionsfunktionen für jede als gut bewertete Messung eines zeitlichen Abschnittes für jede Teilfläche eine Gesamtfunktion für die weitere Auswertung gebildet wird.

2. Verfahren nach Anspruch 1, dadurch gekennzeichnet, daß jeder Schritt der verlängerten m-Sequenz aus einem ersten Teilschritt besteht, in dem die Teilflächen entsprechend der negierten m-Sequenzen hell- und dunkelgesteuert werden und einem zweiten Teilschritt, in dem alle Teilflächen dunkelgesteuert werden, daß die Dauer der beiden Teilschritte einstellbar ist und daß für die Berechnung der Kreuzkorrelationsfunktion der entsprechende Funktionswert der m-Sequenz des ersten Teilschrittes bei jeder Teilfläche für die gesamte Zeitdauer der zu berechnenden Reaktionsfunktion verwendet wird.

3. Verfahren nach Anspruch 1 oder 2, dadurch gekennzeichnet, daß das Signal für die Gesamtreaktion des Auges auf die Überschreitung von Grenzwerten überwacht wird und daß bei Überschreitung der Grenzwerte die m-Sequenzen um eine einstellbare Anzahl von Schritten zurückgesetzt und die Signale für die Gesamtreaktion des Auges durch die wiederholten Werte ersetzt werden.

4. Verfahren nach Anspruch 1, 2 oder 3, dadurch gekennzeichnet, daß zur Bewertung der Qualität jeder Messung mehrere Funktionen aus jeweils einer gleichen Anzahl von Teilflächen gebildet werden, wobei jeder Funktion unterschiedliche, über die Gesamtfläche verteilte Teilflächen zugeordnet sind und daß die Funktionen miteinander und mit den Funktionen vorhergehender oder nachfolgender Messungen verglichen werden und deren Ähnlichkeit als Maß für die Qualität der Messung verwendet wird.

5. Verfahren nach einem der Ansprüche 1 bis 4, dadurch gekennzeichnet, daß jede über einen zeitlichen Abschnitt durchzuführende Messung aus einem vorgelagerten Teilzyklus zum Abklingen von Einschwingvorgängen, aus dem zeitlichen Abschnitt für die Ermittlung des Teilergebnisses und aus einem nachgeordneten Teilzyklus besteht, der mindestens so lang ist, wie die zu berechnende Dauer der Reaktionsfunktion.

6. Verfahren nach einem der Ansprüche 1 bis 5, dadurch gekennzeichnet, daß das Bild in an sich bekannter Weise aus Sechsecken mit von der Mitte nach außen zunehmender Größe gebildet wird.

7. Verfahren nach einem der Ansprüche 1 bis 5, dadurch gekennzeichnet, daß das Bild aus Vierecken mit von der Mitte nach außen zunehmender Größe gebildet wird.

8. Verfahren nach einem der Ansprüche 1 bis 5, dadurch gekennzeichnet, daß das Bild aus Kreisringsegmenten mit von der Mitte nach außen zunehmender Größe gebildet wird.

9. Verfahren nach einem der Ansprüche 1 bis 8, dadurch gekennzeichnet, daß nur Segmente der Fläche zur Darstellung von vorzugsweise kleineren Teilflächen benutzt werden und die anderen Segmente der Fläche über die Dauer der Messung auf eine konstante Helligkeit eingestellt bleiben, um Teile der Netzhaut mit höherer Auflösung zu untersuchen.

10. Verfahren nach einem der Ansprüche 1 bis 9, dadurch gekennzeichnet, daß die Teilflächen mit einer einheitlichen, voreinstellbaren Farbe leuchten.

11. Verfahren nach einem der Ansprüche 1 bis 10, dadurch gekennzeichnet, daß das Signal für die Gesamtreaktion des Auges, das sich aus der Summe der Einzelreaktionen aller Teilflächen zusammensetzt über eine Verstärkung und eine Begrenzung des Frequenzbandes gewonnen wird.

12. Verfahren nach einem der Ansprüche 1 bis 11, dadurch gekennzeichnet, daß die Gesamtreaktion des Auges durch Elektroretinographie ermittelt wird.

13. Verfahren nach einem der Ansprüche 1 bis 11, dadurch gekennzeichnet, daß die Gesamtreaktion des Auges durch Magnetoretinographie ermittelt wird.

14. Verfahren nach einem der Ansprüche 1 bis 11, dadurch gekennzeichnet, daß die Gesamtreaktion des Auges durch die Messung evozierter Potentiale ermittelt wird, die an den entsprechenden Stellen des Kopfes abgenommen werden.

15. Anordnung zur Durchführung des Verfahrens nach Anspruch 1 zur Bestimmung der Topographie für bioelektrische Reaktionssignale eines Auges auf Lichtreize in Abhängigkeit vom Ort des jeweiligen Lichtreizes auf der Netzhaut mit einer vor dem Auge angeordneten Einrichtung (2) zur Darstellung eines aus Teilflächen bestehenden leuchtenden Bildes, die mit einer Steuereinheit (3) verbunden ist, um die Teilflächen hell-und dunkelzusteuern und bei der Mittel vorgesehen sind, um das vom Auge (1) abgenommene Reaktionssignal der Steuereinheit zuzuführen, die weiterhin eine Anzeigeeinheit (4) zur Bedienerführung und Ergebnisdarstellung sowie eine Tastatur (5) zur Bedienung der Anordnung enthält, die beide ebenfalls mit der Steuereinheit (3) verbunden sind und daß die Steuereinheit (3) Mittel zur Erzeugung von vollständigen, für jede Teilfläche um mindestens einen Schritt zyklisch verschobenen m-Sequenzen und Mittel zum Einfügen eines Zusatzschrittes zu einem beliebigen aber für alle m-Sequenzen gleichen Zeitpunkt enthält, und daß in der Steuereinheit (3) weiterhin Mittel zur Berechnung der Kreuzkorrelationsfunktionen aus dem vom Auge (1) abgenommenen Reaktionssignal und den m-Sequenzen vorgesehen sind, mit denen die Reaktionsfunktionen für alle Teilflächen ermittelt werden und daß die Steuereinheit (3) Mittel zur Bewertung der Qualität der Messung nach den Verfahrensansprüchen 1 oder 4 enthält.

**16.** Anordnung nach Anspruch 15, dadurch gekennzeichnet, daß Mittel vorgesehen sind, um die Teilflächen während eines ersten Teilschrittes entsprechend dem Funktionswert der jeweiligen m-Sequenz hell- oder dunkelzusteuern und während eines zweiten Teilschrittes alle Teilflächen dunkelzusteuern und daß die Dauer der beiden Teilschritte einstellbar ist.

**17.** Anordnung nach Anspruch 15 oder 16, dadurch gekennzeichnet, daß Mittel zur Messung und Überwachung des Verlaufes des Signals für die Gesamtreaktion des Auges vorgesehen sind, die bei Überschreitung der Grenzwerte die Mittel zur Erzeugung der m-Sequenzen so beeinflussen, daß alle m-Sequenzen um eine vorgebbare Anzahl von Schritten zurückgesetzt und die fehlerhaften Meßwerte durch die wiederholten Werte ersetzt werden.

**18.** Anordnung nach Anspruch 15, 16 oder 17, dadurch gekennzeichnet, daß die Fläche (2) ein Bildschirm ist.

**19.** Anordnung nach einem der Ansprüche 15 bis 17, dadurch gekennzeichnet, daß die Fläche (2) eine mit Leuchtdioden bestückte Fläche ist.

**20.** Anordnung nach einem der Ansprüche 15 bis 17, dadurch gekennzeichnet, daß die Fläche (2) eine von einem LCD-Projektor bestrahlte Fläche ist.

**21.** Anordnung nach einem der Ansprüche 15 bis 17, dadurch gekennzeichnet, daß die Fläche (2) eine von einem Laser bestrahlte Fläche ist.

**22.** Anordnung nach einem der Ansprüche 15 bis 21 dadurch gekennzeichnet, daß die Signale für die Gesamtreaktion des Auges über einen Verstärker, vorzugsweise einen Differenzverstärker mit über die Steuereinheit (3) einstellbarer oberer und unterer Grenzfrequenz und einer synchron zur Bilddarstellung gesteuerten Abtastschaltung einem Analog-/Digital-Umsetzer in der Steuereinheit (3) zugeführt werden, dessen Ausgangsanschlüsse mit einem Computer zur Weiterverarbeitung verbunden sind und daß in der Steuereinheit (3) Mittel zur Einstellung der Grenzfrequenzen des Verstärkers vorgesehen sind.

**23.** Anordnung nach einem der Ansprüche 15 bis 22, dadurch gekennzeichnet, daß die Steuereinheit (3) Mittel zur Speicherung und zur Darstellung des Zeitverlaufes für die Signale der Gesamtreaktion des Auges enthält.

**24.** Anordnung nach einem der Ansprüche 15 bis 23, dadurch gekennzeichnet, daß die Steuereinheit (3) Mittel zur Darstellung des Zeitverlaufes der über mehrere Teilflächen gebildeten Funktionen enthält.

**25.** Anordnung nach einem der Ansprüche 15 bis 24, dadurch gekennzeichnet, daß die Steuereinheit (3) Mittel zur Darstellung der für die Teilflächen ermittelten Reaktionssignale und deren Amplituden enthält.

**26.** Anordnung nach einem der Ansprüche 15 bis 25, dadurch gekennzeichnet, daß die Steuereinheit (3) mindestens einen Rechner enthält und daß die Erzeugung der digitalen, zyklischen Zeitsignale zur Steuerung des aus Teilflächen bestehenden Bildes, die Auswertung der Meßdaten sowie die Bedienung der Anordnung und die Ergebnisdarstellung über diesen Rechner erfolgt.

**27.** Anordnung nach einem der Ansprüche 15 bis 25, dadurch gekennzeichnet, daß die Steuereinheit (3) zwei Rechner enthält, wobei die Erzeugung der digitalen, zyklischen Zeitsignale zur Steuerung des aus Teilflächen bestehenden Bildes und die Auswertung der Meßdaten über den ersten Rechner erfolgt, der mit dem zweiten Rechner für die Bedienung der Anordnung, die Auswertung und die Ergebnisdarstellung gekoppelt ist.

**Claims**

**1.** Method for determining the topography for bioelectric eye reaction signals relating to light stimuli as a function of the location of the respective light stimulus on the retina, having a surface arranged in front of the eye and on which a luminous image consisting of subsurfaces is displayed for the purpose of stimulation, each subsurface being brightened or darkened as a function of a digital time function assigned to it, and the total reaction of the eye being determined in the process, and in the case of which the component of the reaction function of the eye assigned to the respective subsurface is determined from the total reaction of the eye by calculating the cross-correlation function with the digital time function, characterized in that use is made for the measurement of an arbitrary number, above a minimum value, of identical, inherently closed time intervals which respectively supply a partial result which can be evaluated in that for each time interval use is made as digital time function of a complete m sequence which is displaced cyclically by at least one step for each subsurface and into which an additional step is inserted at an arbitrary instant which is, however, identical for all m sequences, and the reaction function of each subsurface is calculated for each time interval by forming the cross-correlation function, and at least one sum function is formed by summing the reaction functions of a plurality of subsurfaces with which the quality of the measurement of the time interval is evaluated, and for each subsurface a total function is formed for further use in order to reduce the measurement errors by summing the reaction functions for each measurement of a time interval evaluated as good.

2. Method according to Claim 1, characterized in that each step of the lengthened m sequence comprises a first partial step in which the subsurfaces are brightened and darkened in accordance with the negated m sequences, and of a second partial step, in which all the subsurfaces are darkened, in that the duration of the two partial steps can be set, and in that for the purpose of calculating the cross-correlation function the corresponding functional value of the m sequence of the first partial step is used in the case of each subsurface for the total time duration of the reaction function to be calculated.

3. Method according to Claim 1 or 2, characterized in that the signal for the total eye reaction is monitored for exceeding limit values, and in that when the limit values are exceeded the m sequences are reset by an adjustable number of steps, and the signals for the total eye reaction are replaced by the repeated values.

4. Method according to Claim 1, 2 or 3, characterized in that a plurality of functions are formed from an equal number of subsurfaces in each case for the purpose of evaluating the quality of each measurement, each function being assigned different subsurfaces distributed over the total surface, and in that the functions are compared with one another and with the functions of preceding or subsequent measurements and their similarity is used as a measure of the quality of the measurement.

5. Method according to one of Claims 1 to 4, characterized in that each measurement to be carried out over a time interval consists of a preceding partial cycle for the decay of transient phenomena, of the time interval for determining the partial result, and of a succeeding partial cycle which is at least as long as the duration of the reaction function to be calculated.

6. Method according to one of Claims 1 to 5, characterized in that the image is formed in a way known per se from hexagons increasing in size from the centre outwards.

7. Method according to one of Claims 1 to 5, characterized in that the image is formed from rectangles increasing in size from the centre outwards.

8. Method according to one of Claims 1 to 5, characterized in that the image is formed from annular segments increasing in size from the centre outwards.

9. Method according to one of Claims 1 to 8, characterized in that only segments of the surface are used to display preferably smaller subsurfaces, and the other segments of the surface remain set to a constant brightness for the duration of the measurement, in order to examine parts of the retina with higher resolution.

10. Method according to one of Claims 1 to 9, characterized in that the subsurfaces emit light of a single colour which can be preset.

11. Method according to one of Claims 1 to 10, characterized in that the signal for the total eye reaction, which is composed of the sum of individual reactions of all the subsurfaces, is obtained by amplifying and limiting the frequency band.

12. Method according to one of Claims 1 to 11, characterized in that the total eye reaction is determined by electroretinography.

13. Method according to one of Claims 1 to 11, characterized in that the total eye reaction is determined by magnetoretinography.

14. Method according to one of Claims 1 to 11, characterized in that the total eye reaction is determined by the measurement of evoked potentials which are tapped at the corresponding spots on the head.

15. Arrangement for carrying out the method according to Claim 1 for determining the topography for bioelectric eye reaction signals relating to light stimuli as a function of the location of the respective light stimulus on the retina, having a device (2) arranged in front of the eye for displaying a luminous image consisting of subsurfaces, which arrangement is connected to a control unit (3) in order to brighten and darken the subsurfaces, and in the case of which means are provided in order to feed the reaction signal tapped from the eye (1) to the control unit, which furthermore includes a display unit (4) for operation by an operator and for display of results, and also a keyboard (5) for operating the arrangement, both of which are likewise connected to the control unit (3), and in that the control unit (3) includes means for generating complete m sequences displaced cyclically by at least one step for each subsurface, and means for inserting an additional step at an instant which is arbitrary but identical for all m sequences, and in that there are also provided in the control unit (3) means for calculating the cross-correlation functions from the reaction signal tapped from the eye (1) and from the m sequences, with the aid of which the reaction functions are determined for all the subsurfaces, and in that the control unit (3) includes means for evaluating the quality of the measurement according to the Method Claims 1 or 4.

16. Arrangement according to Claim 15, characterized in that means are provided for brightening or darkening the subsurfaces during a first partial step in accordance with the function value of the respective m sequence, and for darkening all the subsurfaces during a second partial step, and in that the duration of the two partial steps is adjustable.

17. Arrangement according to Claim 15 or 16, characterized in that provided for the purpose of measuring and monitoring the profile of the signal for the total eye reaction are means which, when the limit values are exceeded, influence the means for generating the m sequences such that all the m sequences are reset by a prescribable number of steps, and the defective measured values are replaced by the repeated values.

18. Arrangement according to Claim 15, 16 or 17, characterized in that the surface (2) is a display screen.

19. Arrangement according to one of Claims 15 to 17, characterized in that the surface (2) is a surface fitted with light-emitting diodes.

20. Arrangement according to one of Claims 15 to 17, characterized in that the surface (2) is a surface irradiated by an LCD projector.

21. Arrangement according to one of Claims 15 to 17, characterized in that the surface (2) is a surface irradiated by a laser.

22. Arrangement according to one of Claims 15 to 21, characterized in that the signals for the total eye reaction are fed via an amplifier, preferably a differential amplifier with upper and lower cut-off frequencies which can be adjusted by the control unit (3) and with a sampling circuit controlled synchronously with the image display, to an analogue-digital converter in the control unit (3) whose output terminals are connected to a computer for further processing, and in that means are provided in the control unit (3) for setting the cut-off frequencies of the amplifier.

23. Arrangement according to one of Claims 15 to 22, characterized in that the control unit (3) includes means for storing and for displaying the time profile for the signals of the total eye reaction.

24. Arrangement according to one of Claims 15 to 23, characterized in that the control unit (3) includes means for displaying the time profile of the functions formed over a plurality of subsurfaces.

25. Arrangement according to one of Claims 15 to 24, characterized in that the control unit (3) includes means for displaying the reaction signals determined for the subsurfaces and their amplitudes.

26. Arrangement according to one of Claims 15 to 25, characterized in that the control unit (3) includes at least one computer, and in that the generation of the digital, cyclic time signals for controlling the image consisting of subsurfaces, the evaluation of the measured data as well as the operation of the arrangement and the display of results are performed via this computer.

27. Arrangement according to one of Claims 15 to 25, characterized in that the control unit (3) includes two computers,

**EP 0 893 966 B1**

the generation of the digital, cyclic time signals for controlling the image consisting of subsurfaces, and the evaluation of the measured data being performed via the first computer, which is coupled to the second computer for operating the arrangement, the valuation and the display of results.

**Revendications**

1. Procédé pour définir la topographie des signaux de réaction bioélectriques de l'oeil suite à des stimulations lumineuses, en fonction de la localisation sur la rétine de la stimulation lumineuse considérée, à l'aide d'une surface disposée devant l'oeil, sur laquelle on assure la stimulation en représentant une image lumineuse constituée de surfaces partielles, chaque surface partielle étant commandée à l'état éclairé et sombre selon une fonction numérique temporelle correspondante et on mesure ainsi la réaction globale de l'oeil, et avec lequel on obtient les composantes orientées vers la surface partielle correspondante de la fonction de réaction de l'oeil par calcul de la fonction de corrélation croisée avec la fonction numérique temporelle à partir de la réaction globale de l'oeil, caractérisé en ce qu'on utilise pour la mesure, un nombre fini quelconque supérieur à une valeur minimum de sections temporelles identiques, qui fournissent chaque fois un résultat partiel pouvant être traité, en ce qu'on utilise comme fonction numérique temporelle pour chaque élément temporel, une séquence m complète décalée cycliquement d'au moins un pas pour chaque surface élémentaire, dans laquelle on insère un pas supplémentaire à un instant quelconque mais identique pour toutes les séquences m, et qu'on calcule la fonction de réaction de chaque surface partielle en réalisant la fonction de corrélation croisée pour chaque section temporelle, et qu'on élabore au moins une fonction somme par sommation des fonctions de réaction de plusieurs surfaces partielles, en ce qu'il en résulte une évaluation de la qualité de la mesure de la section temporelle et que, pour la réduction des erreurs de mesure, on élabore une fonction globale par sommation des fonctions de réaction de chaque mesure évaluée comme bonne d'une section temporelle pour chaque surface partielle en vue de l'utilisation ultérieure.

2. Procédé selon la revendication 1, caractérisé en ce que chaque pas de la séquence m rallongée est constitué d'un premier pas partiel dans lequel les surfaces partielles correspondant aux séquence m de signe inversé sont commandées pour être éclairées et sombres et un second pas partiel dans lequel toutes les surfaces partielles sont commandées pour être sombres, en ce que la durée des deux pas partiels peut être réglée et en ce que, pour le calcul de la fonction de corrélation croisée, on utilise la valeur de la fonction correspondant à la séquence m du premier pas partiel pour chaque surface partielle sur toute la durée de la fonction de réaction à calculer.

3. Procédé selon la revendication 1 ou 2, caractérisé en ce que, pour la réaction globale de l'oeil, on vérifie le signal vis-à-vis d'un dépassement de valeurs limites et en ce qu'en cas de dépassement des valeurs limites, on rétrograde les séquences m d'un nombre réglable de pas et on remplace les signaux pour la réaction globale de l'oeil par les valeurs qui sont répétées.

4. Procédé selon la revendication 1, 2 ou 3, caractérisé en ce que, pour l'évaluation de la qualité de chaque mesure, on forme plusieurs fonctions chaque fois d'un même nombre de surfaces partielles, des surfaces partielles différentes réparties sur l'ensemble de la surface correspondant à chaque fonction, et en ce qu'on compare les fonctions les unes par rapport aux autres et avec les fonctions des mesures précédentes ou suivantes et dont les ressemblances sont utilisées comme référence pour la qualité des mesures.

5. Procédé selon l'une des revendications 1 à 4, caractérisé en ce que chaque mesure devant être réalisée dans un intervalle de temps donné est constituée d'un cycle partiel de la section temporelle, stocké antérieurement, pour atténuer les phénomènes oscillatoires, pour l'élaboration du résultat partiel et d'un cycle partiel suivant, qui est au moins aussi long que la durée de la fonction de réaction à calculer.

6. Procédé selon l'une des revendications 1 à 5, caractérisé en ce que, de manière connue en soi, l'image est formée d'hexagones de taille croissante du centre vers l'extérieur.

7. Procédé selon l'une des revendications 1 à 5, caractérisé en ce que l'image est formée de quadrilatères de taille croissante du centre vers l'extérieur.

8. Procédé selon l'une des revendications 1 à 5, caractérisé en ce que l'image est formée de secteurs annulaires de taille croissante du centre vers l'extérieur.

9. Procédé selon l'une des revendications 1 à 8, caractérisé en ce que seuls des segments de la surface sont utilisés pour la représentation de surface partielles de préférence plus petites et les autres segments de la surface restent réglés à une luminosité constante pendant la durée de la mesure pour examiner des parties de la rétine avec une résolution supérieure.

10. Procédé selon l'une des revendications 1 à 9, caractérisé en ce que les surfaces partielles rayonnent une lumière de couleur homogène qui peut être préréglée.

11. Procédé selon l'une des revendications 1 à 10, caractérisé en ce que le signal pour la réaction globale de l'oeil, qui résulte de la somme des réactions individuelles de toutes les surfaces partielles, est obtenu à travers une amplification et une limitation de la bande passante.

12. Procédé selon l'une des revendications 1 à 11, caractérisé en ce que la réaction globale de l'oeil est obtenue par électro-rétinographie.

13. Procédé selon l'une des revendications 1 à 11, caractérisé en ce que la réaction globale de l'oeil est obtenue par magnéto-rétinogtaphie.

14. Procédé selon l'une des revendications 1 à 11, caractérisé en ce que la réaction globale de l'oeil est obtenue par la mesure de potentiels évoqués relevés aux emplacements correspondants de la tête.

15. Dispositif pour exécuter le procédé selon la revendication 1 pour définir la topographie des signaux de réaction bioélectriques de l'oeil suite à des stimulations lumineuses, en fonction de la localisation sur la rétine de la stimulation lumineuse considérée, à l'aide d'un équipement (2) disposé devant l'oeil, pour représenter une image lumineuse constituée de surfaces partielles, relié à une unité de commande (3) pour commander les surfaces partielles à l'état éclairé et sombre et dans lequel des moyens sont prévus pour acheminer le signal de réaction provenant de l'oeil (1) vers l'unité de commande, qui contient de plus une unité de visualisation (4) pour la commande par l'utilisateur et pour la représentation des résultats, ainsi qu'un clavier (5) pour la commande du dispositif, qui sont tous deux reliés également à l'unité de commande (3), et que l'unité de commande (3) contient des moyens pour produire des séquences m complètes décalées cycliquement d'au moins un pas pour chaque surface élémentaire, et des moyens pour insérer un pas supplémentaire à un instant quelconque mais identique pour toutes les séquences m, et que dans l'unité de commande (3) sont prévus de plus des moyens pour calculer les fonctions de corrélation croisée à partir du signal de réaction provenant de l'oeil (1) et pour lesquelles des séquences m sont prévues qui permettent de trouver les fonctions de réaction pour toutes les surfaces partielles et que l'unité de commande (3) contient des moyens pour l'évaluation de la qualité de la mesure selon les revendications 1 ou 4 du procédé.

16. Dispositif selon la revendication 15, caractérisé en ce que des moyens sont prévus pour commander pendant un premier pas partiel, les surfaces partielles à l'état éclairé et sombre selon la valeur de la fonction de la séquence m correspondante et pour commander pendant un second pas partiel, toutes les surfaces partielles à l'état sombre et que la durée des deux pas partiels soit réglable.

17. Dispositif selon la revendication 15 ou 16, caractérisé en ce que des moyens sont prévus pour la mesure et la surveillance du déroulement du signal pour la réaction globale de l'oeil qui, en cas de dépassement des valeurs limites, influencent les moyens pour produire les séquences m de telle manière que toutes les séquences m sont rétrogradées d'un nombre de pas prédéfini et que les valeurs de mesures erronées sont remplacées par les valeurs qui sont répétées.

18. Dispositif selon la revendication 15, 16 ou 17, caractérisé en ce que la surface (2) est un écran de visualisation.

19. Dispositif selon l'une des revendications 15 à 17, caractérisé en ce que la surface (2) est une surface équipée de diodes LED.

20. Dispositif selon l'une des revendications 15 à 17, caractérisé en ce que la surface (2) est une surface éclairée par un projecteur à LCD.

21. Dispositif selon l'une des revendications 15 à 17, caractérisé en ce que la surface (2) est une surface éclairée par un laser.

**22.** Dispositif selon l'une des revendications 15 à 21, caractérisé en ce que les signaux pour la réaction globale de l'oeil sont acheminés à travers une amplification, de préférence un amplificateur différentiel avec une fréquence limite supérieure et inférieure réglables à l'aide de l'unité de commande (3) et avec un circuit de lecture commandé de manière synchrone avec la représentation de l'image, un convertisseur analogique - numérique dans l'unité de commande (3) dont les bornes de sortie sont reliées à un ordinateur pour le traitement ultérieur et que des moyens de réglage des fréquences limites de l'amplificateur sont prévus dans l'unité de commande (3).

**23.** Dispositif selon l'une des revendications 15 à 22, caractérisé en ce que l'unité de commande (3) contient des moyens de stockage et de visualisation du déroulement temporel pour les signaux de la réaction globale de l'oeil.

**24.** Dispositif selon l'une des revendications 15 à 23, caractérisé en ce que l'unité de commande (3) contient des moyens de visualisation du déroulement temporel des fonctions formées sur plusieurs surfaces partielles.

**25.** Dispositif selon l'une des revendications 15 à 24, caractérisé en ce que l'unité de commande (3) contient des moyens de visualisation des signaux de réaction trouvés pour les surfaces partielles, et leurs amplitudes.

**26.** Dispositif selon l'une des revendications 15 à 25, caractérisé en ce que l'unité de commande (3) contient au moins un calculateur et que la création des signaux temporels numériques cycliques pour la commande de l'image constituée de surfaces partielles résulte du traitement des données de mesure ainsi que la manipulation du dispositif et la visualisation des résultats au moyen de ce calculateur.

**27.** Dispositif selon l'une des revendications 15 à 25, caractérisé en ce que l'unité de commande (3) contient deux calculateurs, la création des signaux temporels numériques cycliques pour la commande de l'image constituée de surfaces partielles et le traitement des données de mesure résultant du premier calculateur qui est accouplé au second calculateur pour la manipulation du dispositif, le traitement et la visualisation des résultats.

**Fig. 1**

**Fig. 2**

**Fig. 3**

**Fig. 4**

**Fig. 5**

**Fig. 6**